# EUROPEAN PATENT APPLICATION

(11) **EP 4 556 034 A1**
(43) Date of publication of application: **21.05.2025**
(21) Application number: 24164755.1
(22) Date of filing: 20.03.2024
(51) Int. Cl.: A61M 5/20

(54) **IMPROVED RESERVOIR UNIT FOR AN ADMINISTRATION DEVICE**

(71) Applicant: Ypsomed AG, 3401 Burgdorf (CH)
(72) Inventor: Klötzli, Urs, 3414 Oberburg (CH); Scheurer, Simon, 3006 Bern (CH); Hostettler, Patrick, 3415 Hasle (CH); Zumstein, Dominik, 3014 Bern (CH)

(57) **Abstract**

A disposable reservoir unit (1) for an administration device (50), detachably connectable to a drive unit (51) (disposable or reusable) of the administration device (50), the reservoir unit (1) comprising - a holder (2) for an insertable prefilled reservoir (3) - a cover member (4) axially displaceable relative to the holder (2) or relative to the prefilled reservoir (3) - a sensor member (5) wirelessly activatable for read and write operations, wherein the sensor member (5) comprises a wireless tag (21) and a temperature sensor (6) which is thermally couplable to the prefilled reservoir (3) and wherein the sensor member (5) is integrable in one or both of the holder (2) or the cover member (4) by a dedicated receiving cavity (8) adapted to positively fit at least a part of the external shape (7) of the sensor member (5).

## Description

### FIELD OF THE INVENTION

The present invention relates to drug delivery devices or medicament administration devices for injecting, delivering, administering, infusing or dispensing substances and/or liquids such as insulin, hormone preparations or vaccines. It departs from an injection device comprising a reusable drive unit and a disposable reservoir unit configured for releasable attachment to the drive unit.

### BACKGROUND OF THE INVENTION

A variety of diseases exist that require regular treatment by subcutaneous administration of a medicament, and a number of drug delivery devices have been developed to support a patient in accurately and controllably delivering an amount of drug in a self-administration process. Delivery devices include injection devices that are removed from the injection site after each medication event or drug delivery process, as well as infusion devices with a cannula or needle that remains in the skin of the patient for a prolonged period of time.

By way of example, diabetes may be treated by self-administration of insulin or its derivatives with the help of multi-variable-dose insulin injection pens. An injection pen device generally has an elongate device body defining a longitudinal main device axis. An automatic injection device has a motor or a drive spring for biasing a plunger rod and shifting a piston in a container barrel, wherein the drive spring may have to be charged or strained manually prior to injection of a dose. A manually powered delivery drive requires a user to manually provide the energy to move the piston, for instance by applying a distal force component to the injection device.

The medicament dose to be injected may typically be manually selected by turning a dosage knob and observing the actual dialed dose from a dose window or display of the insulin pen. A dose is dispensed by inserting the needle into a suited portion of human skin and by moving the piston manually or by pressing a release button of an automatic injection device. Automatic injection devices may comprise an electronic dose dial mechanism to automatically set a dose.

Also known is the use of autoinjectors with syringes. Autoinjectors usually comprise a body for housing a syringe as well as a drive mechanism in order to move the plunger of the syringe upon actuation of the autoinjector. The drive mechanism typically comprises a source of drive, such as a motor or strong spring for moving a transfer element, for example a rod, which acts on the plunger of the syringe.

For safety and hygiene reasons it is desirable that the needle does not protrude from a housing of the auto injector with the exception of the time when the needle is used for injection of a medicament. Thus, either the autoinjector moves the needle out of the housing for the injection and back into the housing after injection or the housing provides a needle guard which may be moved to unsheathe the needle for injection and which may be moved back to a needle covering position after the injection.

The majority of autoinjectors are configured as single use devices which incorporate both the syringe and the drive mechanism in the same housing. Such devices are usually disposable for hygiene reasons.

Disposable auto injectors comprising an electric actuator or an electronic control require a source of energy which is usually in the form of a battery. However, in this case, the autoinjectors should not be disposed in the regular waste, but have to be subjected to special disposal or to recycling, which is an additional burden to the patient. Further, disposing a battery, motor and/or electronics after a single use is a waste of resources and increases the costs of the autoinjector.

In order to account for a need to handle and dispose the administration device parts differently semi-reusable devices have been developed. Such administration devices typically comprise a reusable drive unit as well as a disposable syringe or cartridge or reservoir unit which may be releasably coupled to the drive unit. The drive unit usually includes the drive mechanism and electronics whereas the reservoir unit includes the syringe with the needle and a needle cover sleeve. The user can thus discard the reservoir unit when it is empty or after use and can load the drive unit with a new reservoir unit for the upcoming injection.

Prior art administration devices may include a temperature measurement system and/or a identification system such that a unit of the device may process the information of the drug that is received from the tag sensor of the identification system, and based on the processed information, determine the temperature of the drug within a reservoir.

WO16140853 A1 discloses devices which include a transponder for determining a characteristic of a dose delivery device. The transponder may include a first sensor configured to detect a first parameter of the dose delivery device based on movement of at least one of the dose delivery device or a medicament contained within the dose delivery device, wherein the first sensor is configured to generate a first signal indicative of the first parameter. The transponder may include a processor operably coupled to the first sensor and configured to process the first signal. The transponder may include an indicator unit operably coupled to the processor and configured to generate an output based on the first signal, wherein the output is perceivable by a user. The transponder may also include a battery configured to provide power to at least one of the first sensor, the processor, and the indicator unit.

WO16033507 A2 discloses systems for drug delivery devices include a temperature control system and an identification system. A temperature control system configured to sense and control temperature of a cartridge containing a drug includes a heater, one or more temperature sensors, and a control unit. An identification system configured to identify a cartridge containing a drug includes a control unit and a tag sensor that is electrically coupled to the control unit, wherein the tag sensor is activated upon detecting a presence of the cartridge. A drug delivery device includes both the temperature control system and the identification system such that the control unit of the device may process the information of the drug that is received from the tag sensor of the identification system and based on at least a portion of the processed information, determine and control the temperature of the drug within a cartridge.

WO16115372 A1 describes a smart module configured to work with injector devices. The smart module may include electronics such as sensors and indicators and are programmed to detect environmental parameters. If the sensors detect that a parameter is outside of a predetermined boundary or range, then the indicators may be activated to notify the user that the injector device may not be safe for administering medicine.

### DESCRIPTION OF THE INVENTION

It an objective of the invention to improve the safety in handling or use of reservoir units or of administration devices including reservoir units respectively. It is a further objective of the invention to provide a simplified manufacturing and assembly and to reduce waste for automatic administration devices.

This objectives are achieved by a disposable reservoir unit or an administration device according to the independent claims. Variants of embodiments of the invention are evident from the dependent claims and this description.

The invention relates to a disposable reservoir unit for an administration device, detachably connectable to a disposable or reusable drive unit of the automatic administration device, the reservoir unit comprising:
- a holder for an insertable prefilled reservoir
- a cover member axially displaceable relative to the holder or relative to the prefilled reservoir
- a sensor member wirelessly activatable for read and write operations
wherein the sensor member comprises a wireless tag and a temperature sensor which is thermally couplable to the prefilled reservoir and wherein the sensor member is integrable in one or both of the holder or the cover member by a dedicated receiving cavity adapted to positively fit at least a part of the external shape of the sensor member. By way of example the dedicated receiving cavity can be formed as a box or a trench or a bore or a blind hole or a pocket with form fit or locking fit or snap fit capabilities.

In a variant embodiment of the invention the finally assembled reservoir unit does not comprise the sensor member in the receiving cavity.

The intended use and save and compliant administration of modern medicaments needs the value of certain physical or chemical parameters of the medicament in range. Temperature of the medicament is crucial for most of these parameters e.g. viscosity, activity, dispersibility or absorbability. The precise and sound placement of a sensor member close by the medicament volume is enabled and eased with the dedicated receiving cavity according to the invention.

Besides that, a disposable reservoir unit may be assembled or used without a sensor member inserted in the receiving cavity to reduce the handling and tooling effort in production for cost sensitive platform variants or environmentally friendly applications.

The disposable reservoir unit is part of the semi-disposable or semi-reusable administration device. The reservoir unit is hence not operational without a reusable drive unit of the administration device. To prepare the administration device for an administration the user must attach or connect the reservoir unit to a drive unit. The drive unit may include drive means adapted to dispense the liquid drug from the reservoir hold inside the reservoir unit once the reservoir unit is attached to the drive unit.

The administration device may be a manual or an automatic administration device e.g. an automatic injection device or an autoinjector or an autopen. Automatic devices typically include automatic drive means such as an elastic element (for example a pre-tensioned spring) or an electric motor to drive a dispensing member to dispense the liquid drug form the reservoir e.g. from a prefilled syringe or from a prefilled cartridge both providing a barrel containing the medicament and a moveable stopper as a dispensing member. Manual injection devices include a manual drive such as a dispensing button or a plunger rod that has to be pressed by the user to move the dispensing member in dispensing direction.

The cover member or needle cover is movable along the longitudinal axis and guided by the reservoir holder or holder. The cover member may be implemented as shield or sleeve, or as sleeve-shaped element adapted to cover or envelop a needle of a prefilled syringe or a needle hub and septum of a prefilled cartridge mounted inside the reservoir holder. The movement of the needle cover allows to switch between a safety state in which the needle or needle hub is covered and the needle does not protrude from a device housing and an injection state in which the needle is uncovered and exposes or protrudes from the housing.

In an embodiment the cover member is the outermost part of the disposable reservoir unit forming part of the device housing. That means the reservoir unit does not comprise any outer housing or shell but only the movable cover member. This provides for a simple design. In an alternative embodiment a label is provided on the cover member.

The holder, the cover member and the cap may be plastic parts. The term "separate" means that before assembly the parts can be handled independently from each other and may be produced separately and independently from other parts. That does not exclude that the parts may be connected upon assembly of the reservoir unit. The parts may be connected to each other, for example, by a snap-fit or a thread and/or linear guiding structures.

In the present context, the terms "substance", "drug", "medicament" and "medication" are to be understood to include any flowable medical formulation suitable for controlled administration through a means such as, for example, a cannula or a hollow needle, and comprises a liquid, a solution, a gel or a fine suspension containing one or more medical active ingredients. A medicament can be a composition comprising a single active ingredient or a pre-mixed or co-formulated composition with more than one active ingredient present in a single container. Medication includes drugs such as peptides (e.g., insulin, insulin-containing drugs, GLP-1 containing drugs or derived or analogous preparations), proteins and hormones, active ingredients derived from, or harvested by, biological sources, active ingredients based on hormones or genes, nutritional formulations, enzymes and other substances in both solid (suspended) or liquid form but also polysaccharides, vaccines, DNA, RNA, oligonucleotides, antibodies or parts of antibodies but also appropriate basic, auxiliary and carrier substances

The term "distal" is meant to refer to the direction or the end of the drug delivery device carrying an injection needle or an injection cannula, whereas the term "proximal" is meant to refer to the opposite direction or end pointing away from the needle or cannula.

The term "injection system" or "injector" refers to an administration device that is removed from the injection site after each medication event or drug delivery process, whereas the term "infusion system" refers to a device with a cannula or needle that remains in the skin of the patient for a prolonged period of time, for example, several hours.

In the claims, the word "comprising" does not exclude other elements or steps, and the indefinite article "a" or "an" does not exclude a plurality. For example "an arm" does not exclude the fact that there may be two arms that functionally or structurally fulfill the purpose of "an arm". The mere fact that certain elements or steps are recited in distinct claims shall not preclude the existence of further meaningful combinations of these elements or steps.

The sensor member can be built integrally with a flat or cylindrical substrate or mount, for example as a printed circuit with an adhesive film or as an integrated circuit encapsulated with a glass or resin body for instance a cylindrical glass or epoxy body with an e.g. circular cross section and spherical ends.

The substrate or mount allow for a cost-effective and precise and loss-proof placing of the sensor member in the receiving cavity for instance in case of a high volume production or in case of pre production batches for platform variant devices. Furthermore, the encapsulation or mount protects the circuit from damage or environmental influences.

The wireless tag is integrated in the sensor member or the wireless tag is separate from the rest of the sensor member and connected by a wiring.

These arrangements allow for optimized thermal coupling of the temperature sensor with respect to the reservoir barrel and for optimized radio frequency exposition of the wireless tag.

The sensor member is integrated by the dedicated receiving cavity by a positive fit alone and/or by one or more of an adhesive process, a welding process e.g. a welding between or under one or more protective layers, press-fitting, clamping, snapping or overmolding.

This enables a captive integration of the sensor member combined with a defined thermal conductivity and loss-proof assembly steps.

The local integration and positioning of the sensor member in or on the reservoir unit is ensured by the receiving cavity adapted to the external shape of the sensor member, the receiving cavity and/or the external shape comprising one or more of a depression, deepening, recess, pin, cam, surface, rib, bore, slot, clamp on or in one or more of the holder, the cover member or the sensor member. The external shape of the sensor member may for instance be complementary to the internal shape of the receiving cavity.

These mutual adaptations allow for precise placement and orientation during assembly and use of the sensor member with respect to the barrel of the reservoir.

The integration of the sensor member in one or both of the holder or the cover member may take place before or during or after the assembly of the reservoir unit.

These alternatives allow for straightforward assembly steps and easy supervision of the assembly process. Due to the captive fit of the sensor member in the receiving cavity the sensor member sits loss-proof in the unassembled or preassembled parts e.g. the holder or the cover member.

The thermal coupling of the temperature sensor to the inserted reservoir may be improved by elastically effected contact pressure and/or gap-free contact of the holder or the cover member around the receiving cavity to the surface of the reservoir.

A shorter measurement delay and a better noise tolerance of the measured temperature values can be achieved.

Alternatively, or additionally the thermal coupling of the temperature sensor to the holder or the cover member or the inserted reservoir is increased by thermally conductive paste filled in the spaces formed in between and around the receiving cavity and the external shape of the sensor member or by a thermally conductive film or molded body or by thermally conductive additives in the injection molding plastic.

A shorter measurement delay and a better noise tolerance of the measured temperature values can be achieved.

The cover member is a sleeve in a preferred embodiment of the reservoir unit. The holder is then concentrically arranged inside the sleeve. Therefore, the cover member is movable along the longitudinal axis outside and relative to the holder if the cover member is not blocked or hold in the covering position. The reservoir unit may further include a cap and or a label or a prefilled reservoir.

The sleeve form allows for a compact design.

In an embodiment the thermal coupling of the temperature sensor to the inserted reservoir takes place at or within an axial section of the prefilled reservoir which contains medication.

Shorter measurement delay and better noise tolerance of the measured temperature values can be achieved.

In an embodiment the insertable or inserted prefilled reservoir includes at a distal end section a non-detachably connected cannula e.g. a staked needle or a penetrable septum e.g. hold by a crimp collar.

Furthermore, the insertable or inserted prefilled reservoir includes a releasably tightly closing sleeve e.g. a rigid needle shield "RNS" at the distal end section, which completely encloses the distal end section or the cannula.

The sensor member in an embodiment of the reservoir unit can be activated, written to, or read out wirelessly by a read-write unit.

The invention relates further to an administration device including the reservoir unit e.g. a disposable reservoir unit as described above and a drive unit e.g. a reusable drive unit. The latter may include a plunger rod and a drive for moving the plunger rod in a dispensing direction to dispense the drug from the reservoir e.g. from a syringe including a plug e.g. a rubber stopper. The reservoir unit may be releasably attachable to the drive unit.

As mentioned above the administration device may be a manual injection device requiring a user force to drive the plunger rod to dispense the drug or alternatively the injection device may be an automatic injection device including an automatic drive, for example, in form of a biased spring member or an electric motor. The administration device may be a semi-reusable (or semi-disposable) autoinjector. The automatic drive may comprise an electric motor adapted to drive the plunger rod to dispense the drug from the syringe.

The drive unit of the administration device may further include the read-write unit. Additionally or alternatively an external device such as a remote control, an add on device or a smartphone includes the read-write unit. The drive unit may include a controller, a display and a button.

In an embodiment the cover member may be locked to the holder in the covering position, and the cover member may be unlocked by relative movement between the reservoir unit and the drive unit. The reservoir unit may be moved manually by a user or automatically by the automatic drive of the drive unit.

The scope of the invention includes the use of a reservoir unit according to an above variant of an embodiment of the invention to build an administration device. The invention may further include the use of a prefilled reservoir and/or a drive unit and or/a cap to build the administration device.

### BRIEF DESCRIPTION OF THE DRAWINGS

The subject matter of the invention will be explained in more detail in the following text with reference to preferred exemplary embodiments which are illustrated in the attached drawings, in which:
- Fig. 1: depicts an exploded perspective view of a first embodiment of a disposable reservoir unit according to the invention;
- Fig. 2a: depicts a proximal and a lateral view of the disposable reservoir unit from figure 1;
- Fig. 2b: depicts a sectional view (C-C) of the disposable reservoir unit from figure 2a;
- Fig. 2c: depicts a sectional view (D-D) of the disposable reservoir unit from figure 2a;
- Fig. 3: depicts an exploded perspective view of a second embodiment of a disposable reservoir unit with an embodiment of a sensor member;
- Fig. 4a: depicts a lateral view of the disposable reservoir unit from figure 3 in a preassembled state;
- Fig. 4b: depicts a perspective view of the disposable reservoir unit from figure 3 in a preassembled state;
- Fig. 5a: depicts a proximal, a lateral and a cross sectional view (K-K) of the disposable reservoir unit from figure 3;
- Fig. 5b: depicts a sectional view (J-J) of the disposable reservoir unit from figure 5a;
- Fig. 5c: depicts a sectional view (L-L) of the disposable reservoir unit from figure 5a with a receiving cavity and the sensor unit;
- Fig. 6: depicts an exploded perspective view of parts of further embodiments of disposable reservoir units showing alternative mounting locations of a sensor member;
- Fig. 7a: depicts a perspective view of an administration device with an external device;
- Fig. 7b: depicts a perspective partial section view of the administration device from figure 7a.

The reference symbols used in the drawings, and their primary meanings, are listed in summary form in the list of designations. In principle, identical parts or aequivalent functional units are provided with the same reference symbols in the figures.

### DETAILED DESCRIPTION OF PREFERRED EMBODIMENTS

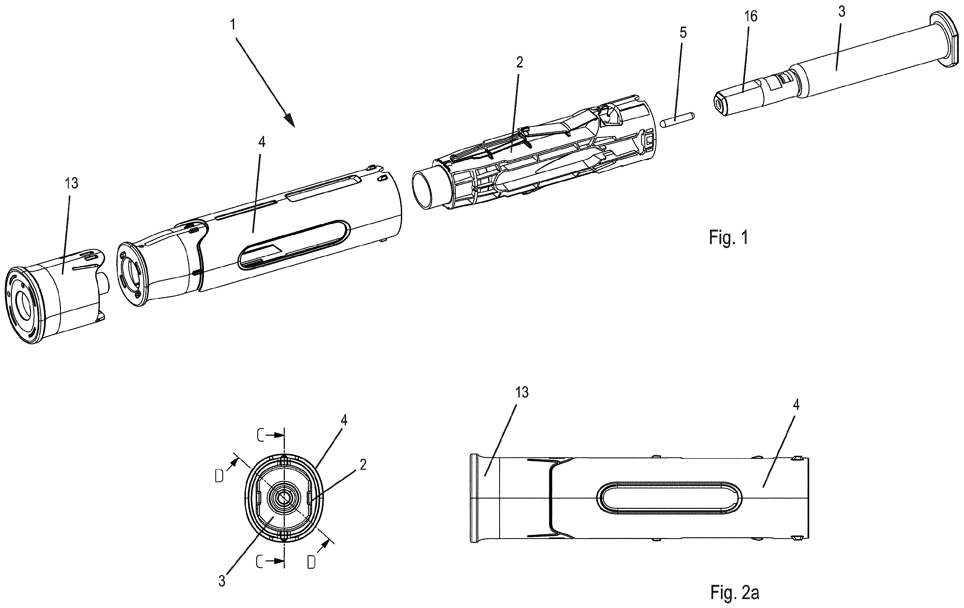

In the present description the term "distal" refers to the side where the injection needle is located. This is on the left-hand side in the figures 1 to 7b. The term "proximal" refers to the opposite side or rear end of the device and is on the right-hand side in the figures. In the following the structural features and parts of the reservoir unit 1 and the administration device 50 according to the invention will be descripted exemplarily. Subsequently, the function of the semi-reusable administration device 50 will be explained.

Figure 1 shows a perspective view of a first embodiment of a disposable reservoir unit 1. A holder 2 is adapted to coaxially hold a reservoir in the form of a prefilled syringe 3 with a needle sleeve 16 in the form of a rigid needle shield. A sensor member 5 is built integrally with a substrate or mount, including an integrated circuit protected in a cylindrical glass body with a circular cross section and spherical caps. A cover member 4 is adapted to coaxially house and axially guide the holder 2 and the reservoir 3 respectively. A cap 13 is detachable from the distal end of the cover member 4 and adapted to remove the needle sleeve.

In the proximal view Figure 2a shows the reservoir unit 1 from figure 1. The prefilled reservoir 3 is inserted in the holder 2 and the cover member 4. In the lateral view figure 2a shows the reservoir unit 1 with the cap 13 attached at the distal end of the cover member 4.

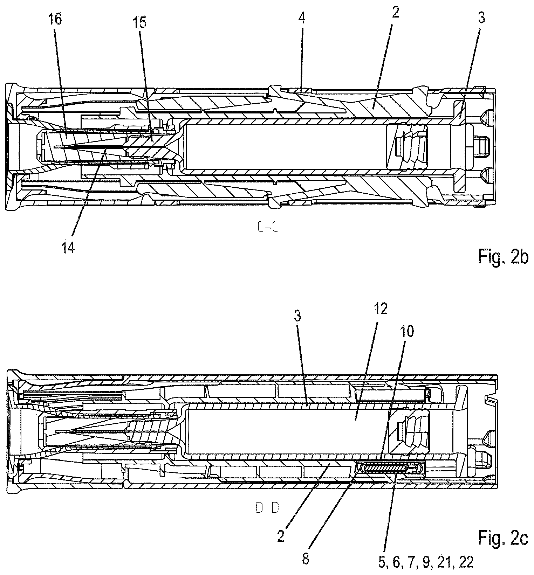

Figure 2b shows a sectional view C-C as labelled in figure 2a. It shows the prefilled reservoir 3 with a distal end section 15 and a cannula 14 staked therein. The reservoir 3 is assembled in the holder 2. The cover member 4 ist kept axially movable on the holder 2.

Figure 2c shows a sectional view D-D as labelled in figure 2a. It shows the prefilled reservoir 3 containing medication 12 in its interior. A receiving cavity 8 in form of a blind hole or pocket is formed in the holder 2. The receiving cavity 8 adjoins the outer surface 10 of the reservoir 3. The sensor member 5 in a glass body 22 with an external shape 7 is embedded in the receiving cavity 8 and such thermally closely coupled with the medication 12 in the prefilled reservoir 3. In its interior the sensor member 5 houses a circuit comprising a wireless tag 21 and a temperature sensor 6.

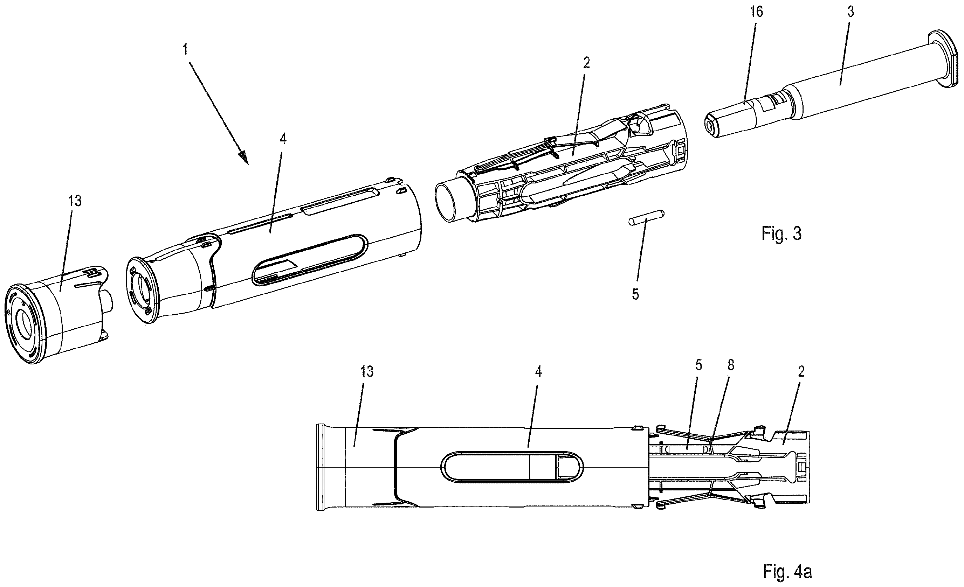

Figure 3 depicts a perspective view of a second embodiment of a disposable reservoir unit 1. A holder 2 is adapted to coaxially hold a reservoir in the form of a prefilled syringe 3 with a needle sleeve 16 in the form of a rigid needle shield. A sensor member 5 is built integrally with a substrate or mount, including an integrated circuit protected in a cylindrical glass body with a circular cross section and spherical caps. A cover member 4 is adapted to coaxially house and axially guide the holder 2 and the reservoir 3 respectively. A cap 13 is detachable from the distal end of the cover member 4 and adapted to remove the needle sleeve.

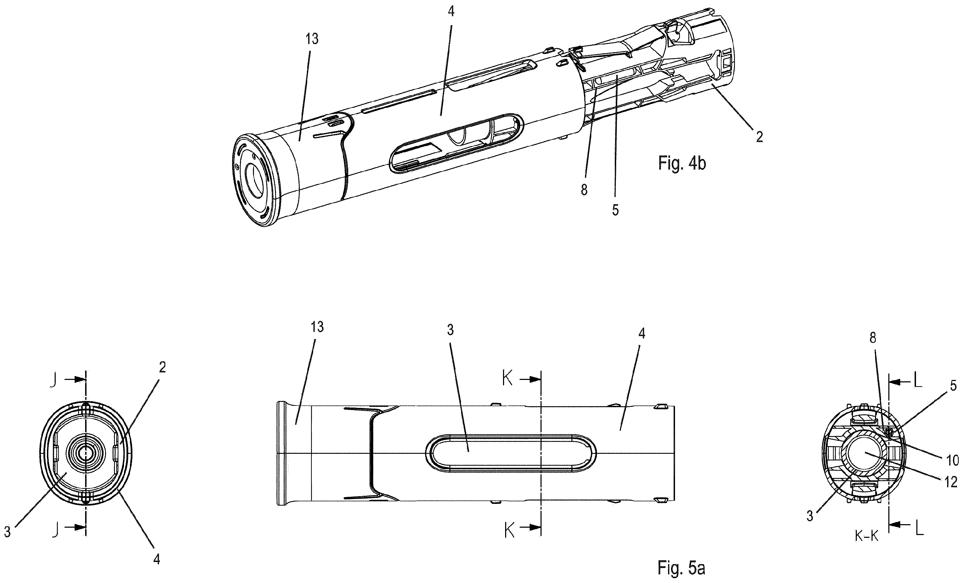

In a lateral view figure 4a shows the reservoir unit 1 from figure 3 in a preassembled state with the cap 13 attached at the distal end of the cover member 4, the holder 2 in an intermediate position coaxially inserted in the cover member 4. The sensor member 5 is inserted in the receiving cavity 8. The receiving cavity 8 having the shape of a box or a trench molded by or in the wall of the holder 2.

In a perspective view figure 4a shows the reservoir unit 1 from figure 4a in a preassembled state with the cap 13 attached at the distal end of the cover member 4, the holder 2 in an intermediate position coaxially inserted in the cover member 4. The sensor member 5 is inserted in the receiving cavity 8. The receiving cavity 8 having the shape of a box or a trench molded by or in the wall of the holder 2.

In the proximal view Figure 5a shows the reservoir unit 1 from figure 3. The prefilled reservoir 3 is inserted in the holder 2 and the cover member 4. In the lateral view figure 5a shows the reservoir unit 1 with the cap 13 attached at the distal end of the cover member 4. The prefilled reservoir 3 is visible through a window in the cover member 4. In the cross sectional view K-K of figure 5a the sensor member 5 is held in the receiving cavity 8. The receiving cavity 8 is thermally coupled to the surface 10 of the syringe 3 with the medication 12 in its interior.

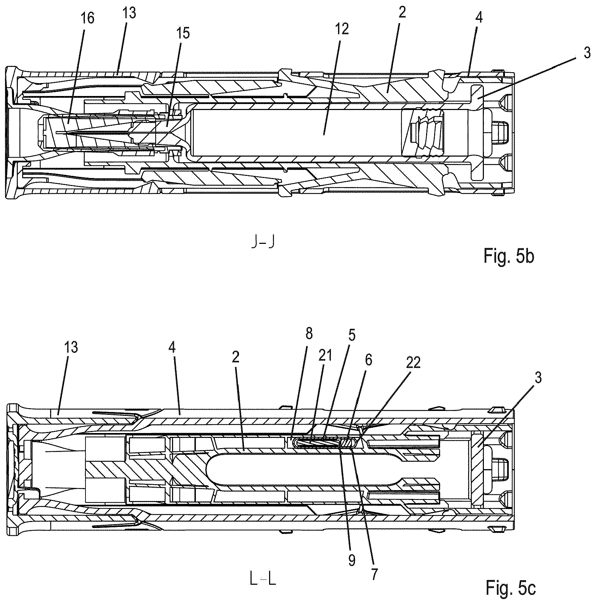

Figure 5b shows a sectional view J-J as labelled in figure 5a. It shows the inserted prefilled reservoir 3 containing medication 12 and a needle sleeve 16 sealingly mounted on the distal end section 15 of the prefilled reservoir 3. The needle sleeve 16 is operatively coupled to the cap 13 attached to the cover member 4. The prefilled reservoir 3 is held coaxially within the holder 2.

Figure 5c shows a sectional view L-L as labelled in figure 5a. It shows the cover member 4 with the cap 13 attached and the holder 2 with the prefilled reservoir 3 inserted. In the holder 2 the receiving cavity 8 is visible with the sensor member 5 inserted or captured respectively. The sensor member 5 includes a wireless tag 21 e.g. a RFID circuit and a temperature sensor 6 mounted on substrate 9 e.g. a printed circuit board. This arrangement is sealed in a glass body 22 with an external shape 7.

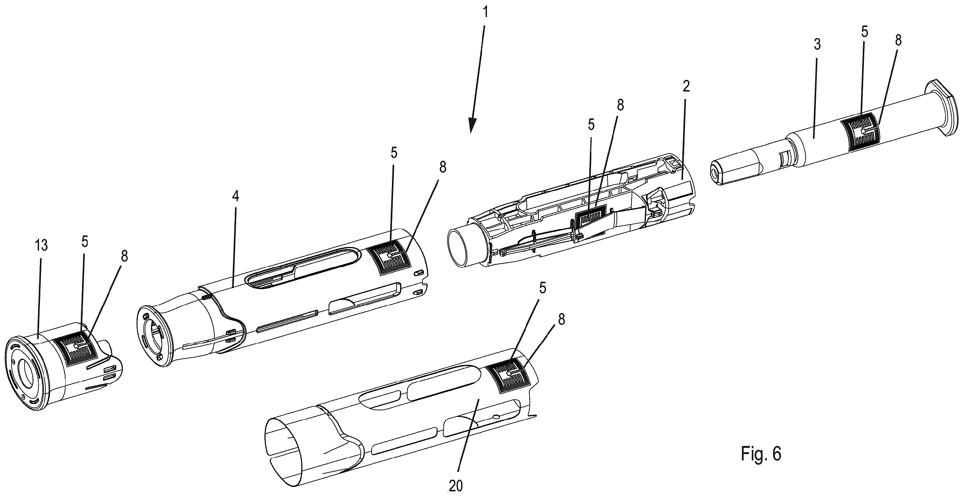

Figure 6 depicts an exploded perspective view of further embodiments or examples of the reservoir unit 1. Placement options for a sensor member 5 formed exemplarily as a rectangular substrate attached in a adapted receiving cavity 8 e.g. a deepening for the cap 13, the cover member 4, the holder 2, an outer label 20 and the reservoir unit 3 are shown.

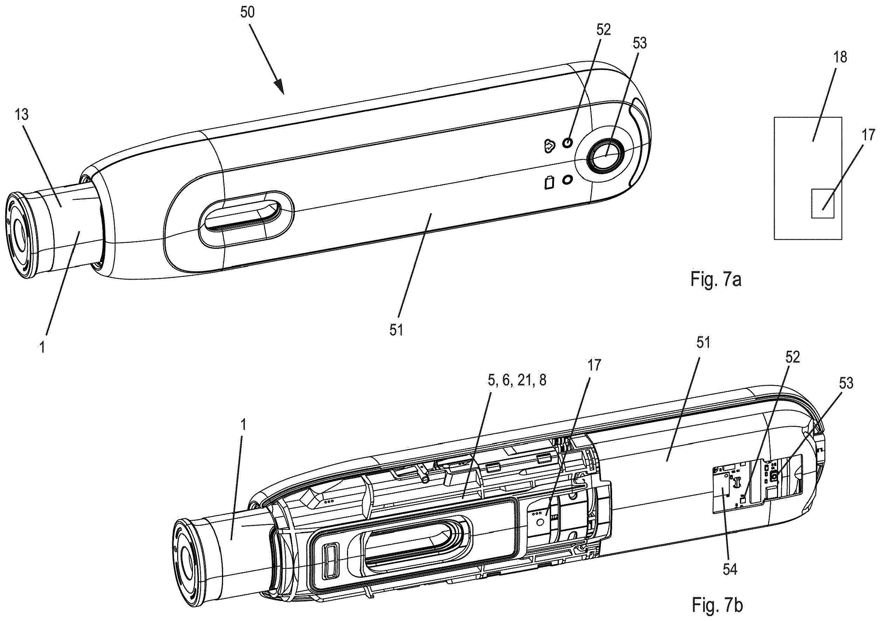

Figure 7a depicts the autoinjector 50 in an assembled state with a reservoir unit 1 attached to the drive unit 51 of the autoinjector 50. The reservoir unit 1 is inserted and thus a position sensor detects the movement and the controller 54 in the electronic module detects the presence of the reservoir unit 1 inside the drive unit 51. A wireless code reader (not shown) inside the drive unit 51 reads the RFID code and the temperature of the reservoir unit 1. Subsequently, the controller 54 of the drive unit 51 may send the read medication information from the read code and/ or the measured temperature value to an external device 18 to verify the drug or medication with predefined information. Alternatively, the controller 54 in the drive unit 51 compares the read medication information from the code with predefined information stored in a memory inside the drive unit 51. In a variant a read-write unit 17 provided in an external device 18 e.g. in a smartphone, may directly perform read-write operations from/to the sensor member 5 in the reservoir unit 1.

In case of successful medication verification, the controller 54 receives an enable signal (either from the external device 18 or from the internal comparison). The controller 54 then controls an electric motor to rotate a threaded rod to move a trigger sleeve in the drive unit 51 a short distance of several millimeters in proximal direction and to lock the reservoir unit 1 in the drive unit 51.

The controller 54 displays the locked state or the unlocked state resp. to the user e.g. on the external device 18 or on a LED 52 on the drive unit 51of the autoinjector 50.

As in this state the cap 13 is still mounted onto the distal end of the cover member 4. The next step is the decapping which may be started by pressing the button 53. The controller 54 then drives the motor to move the reservoir unit 1 back and forth. Consequently, the cap 13 is wiped off. Alternatively, the trigger sleeve proximal movement in the drive unit 51 releases a cap lock of the syringe unit 1 meaning that the cap 13 is no longer locked to the syringe unit 1 and the user can remove the cap 13 manually.

The autoinjector 50 is now decapped and ready for an injection. The controller 54 displays a corresponding notification on the display 52 and/or on the external device 18.

As a next step the user places the distal end of the cover sleeve 4 onto an injection site and pushes the autoinjector 50 towards the injection site. This causes the cover member 4 to move proximally from the distal covering position into the drive unit housing (push on skin). This further movement is detected by a position sensor which triggers the controller 54 in the electronic module to start the injection. That means the controller 54 drives the electric motor in dispensing direction and thus rotates the threaded rod and thereby moves the plunger rod in distal direction. The distal flange of the plunger rod moves the piston inside the prefilled reservoir 3 in distal direction and thus dispenses liquid drug through the cannula 14 out of the prefilled reservoir 3.

The controller 54 displays a holding time indicating a time period during that the user has to hold the autoinjector 50 onto the injection side after the injection to ensure that the drug is completely administered and absorbed. The controller 54 may display a down counter informing the user about the remaining holding time. Once the holding time is elapsed a notification is shown to the user that the autoinjector can be removed from the injection site. Alternatively, the end of the holding time may be indicated only by a LED.

When the user removes the autoinjector 50 from the injection site the cover member 4 moves back distally into the needle covering position.

Furthermore, the entire reservoir unit 1 can be pulled out of the drive unit 51 by the user. The user can now discard and/or recycle the reservoir unit 1 and the drive unit 3 is ready to be loaded with a new reservoir unit 1.

Figure 7b depicts a perspective partial section view of the autoinjector 50 from figure 7a in a locked state with a cap 13. The reservoir unit 1 is inserted and the designators 5,6,21,8 point to the area of the housing part of the drive unit 51 covering the sensor member 5, the temperature sensor 6, the wireless tag 21 and the receiving cavity 8. Beside the controller 54 with the internal elements of the button 53 and the display 52 is the read-write unit 17 visible.

While the invention has been described in detail in the drawings and foregoing description, such description is to be considered illustrative or exemplary and not restrictive. Variations to the disclosed embodiments can be understood and effected by those skilled in the art and practising the claimed invention, from a study of the drawings, the disclosure, and the appended claims.

**LIST OF DESIGNATIONS**

| | | | |
|---|---|---|---|
| 1 | reservoir unit | 50 | administration device |
| 2 | holder | 51 | drive unit |
| 3 | prefilled reservoir | 52 | display |
| 4 | cover member | 53 | button |
| 5 | sensor member | 54 | controller |
| 6 | temperature sensor | | |
| 7 | external shape | | |
| 8 | receiving cavity | | |
| 9 | substrate | | |
| 10 | surface | | |
| 11 | axial section | | |
| 12 | medication | | |
| 13 | cap | | |
| 14 | cannula | | |
| 15 | distal end section | | |
| 16 | needle sleeve | | |
| 17 | read-write unit | | |
| 18 | external device | | |
| 20 | label | | |
| 21 | wireless tag | | |
| 22 | glass body | | |
| 23 | septum | | |

## Claims

1. A disposable reservoir unit (1) for an administration device (50), detachably connectable to a drive unit (51) of the administration device (50), the reservoir unit (1) comprising:
- a holder (2) for an insertable prefilled reservoir (3)
- a cover member (4) axially displaceable relative to the holder (2) or relative to the prefilled reservoir (3)
- a sensor member (5) wirelessly activatable for read and write operations
**characterized in that**
the sensor member (5) comprises a wireless tag (21) and a temperature sensor (6) which is thermally couplable to the prefilled reservoir (3) and wherein the sensor member (5) is integrable in one or both of the holder (2) or the cover member (4) by a dedicated receiving cavity (8) adapted to positively fit at least a part of the external shape (7) of the sensor member (5).

2. The disposable reservoir unit (1) according to claim 1, wherein the sensor member (5) can be built integrally with a flat or cylindrical substrate (9), for example as a printed circuit with an adhesive film or as an integrated circuit with a glass body (22).

3. The disposable reservoir unit (1) according to claim 1 to 2, wherein the wireless tag (21) is integrated in the sensor member (5) or the wireless tag (21) is separate from the rest of the sensor member (5) and connected by a wiring.

4. The disposable reservoir unit (1) according to any of claims 1 to 3, wherein the sensor member (5) is integrated by the dedicated receiving cavity (8) by a positive fit alone and/or by one or more of an adhesive process, a welding process, press-fitting, clamping, snapping or overmolding.

5. The disposable reservoir unit (1) according to any of claims 2 to 4, wherein the local integration and positioning of the sensor member (5) in or on the reservoir unit (1) is ensured by the receiving cavity (8) adapted to the external shape (7) of the sensor member (5), the receiving cavity (8) and/or the external shape (7) comprising one or more of a depression, recess, pin, cam, surface, rib, bore, slot, clamp on or in one or more of the holder (2), the cover member (4) or the sensor member (5).

6. The disposable reservoir unit (1) according to any of claims 1 to 5, wherein the integration of the sensor member (5) may take place before the assembly of the reservoir unit (1).

7. The disposable reservoir unit (1) according to any of claims 1 to 6, wherein the thermal coupling of the temperature sensor (6) to the inserted reservoir (3) is ensured by elastically effected contact pressure and/or gap-free contact of the holder (2) or the cover member (4) around the receiving cavity (8) to the surface (10) of the reservoir (3).

8. The disposable reservoir unit (1) according to any of claims 1 to 7, wherein the thermal coupling of the temperature sensor (6) is increased by thermally conductive paste, film, molded body or by thermally conductive additives in the injection molding material.

9. The disposable reservoir unit (1) according to any of claims 1 to 8, wherein the reservoir unit (1) further comprises an inserted reservoir (3) and a cap (13).

10. The disposable reservoir unit (1) according to claim 9, wherein the thermal coupling of the temperature sensor (6) to the inserted reservoir (3) takes place at an axial section (11) of the prefilled reservoir (3) which contains medication (12).

11. The disposable reservoir unit (1) according to any of claims 9 or 10, wherein the inserted reservoir (3) comprises at a distal end section (15) a non-detachably connected cannula (14) or a penetrable septum (23).

12. The disposable reservoir unit (1) according to any of claims 9 to 11, wherein the inserted reservoir (3) comprises a releasably tightly closing sleeve (16) at the distal end section (15), which completely encloses the distal end section (15) or the cannula (14).

13. The disposable reservoir unit (1) according to any of claims 1 to 12, wherein the sensor member (5) can be activated, written to or read out wirelessly by a read-write unit (17).

14. The disposable reservoir unit (1) according to claim 13, wherein the drive unit (51) of the administration device (50) includes the read-write unit (17) and/or wherein an external device (18) such as a remote control, an add on device or a smartphone includes the read-write unit (17).

15. Use of a disposable reservoir unit (1) according to any of claims 1 to 14 to build an administration device (50).
